# DEMANDE DE BREVET EUROPEEN

(11) **EP 4 737 005 A1**
(43) Date de publication de la demande: **06.05.2026**
(21) Numéro de dépôt: 25201937.7
(22) Date de dépôt: 12.09.2025
(51) Int. Cl.: B01L 3/00, B01L 7/00, C12Q 1/68

(54) **CARTOUCHE MICROFLUIDIQUE DISPOSANT D'UN INSERT THERMIQUEMENT CONDUCTEUR**

(30) Priorité: 30.10.2024 FR 2411877
(71) Demandeur: Commissariat à l'Energie Atomique et aux Energies Alternatives, 75015 Paris (FR)
(72) Inventeur: FOUILLET, Yves, 38054 Grenoble cedex 09 (FR); PARENT, Charlotte, 38054 Grenoble cedex 09 (FR)
(74) Mandataire: INNOV-GROUP

(57) **Abrégé**

L'invention concerne une cartouche microfluidique (1) comportant plusieurs couches superposées et assemblées entre elles, lesdites plusieurs couches comportant :
- Une première couche fluidique, dite couche supérieure (10), comprenant un circuit microfluidique, le circuit microfluidique comportant au moins une face, dite inférieure, dans laquelle est réalisée une cavité (20) formant un volume interne ;
- Une deuxième couche comportant une membrane (11) assemblée sur la première couche et venant refermer ladite cavité (20) pour former une capsule microfluidique ;
- Une troisième couche, dite couche inférieure (12), comportant un circuit d'actionnement de ladite membrane, ledit circuit d'actionnement comportant au moins un passage traversant réalisé à l'aplomb de ladite cavité (20),
- Ladite cartouche comportant un élément formant un insert (4) venant se loger dans ledit passage traversant, et disposant d'au moins une zone de contact avec ladite membrane (11), cet insert (4) étant réalisé dans un matériau conducteur thermique.

## Description

### Domaine technique de l'invention

La présente invention se rapporte à une cartouche microfluidique disposant d'un insert thermiquement conducteur. L'invention concerne également un dispositif microfluidique comportant ladite cartouche microfluidique et un procédé de commande du dispositif microfluidique.

### Etat de la technique

Il est connu du brevet EP3541514B1 de réaliser une cartouche microfluidique, également appelée carte microfluidique ou puce microfluidique, comprenant une ou plusieurs capsules microfluidiques connectées entre elles au sein d'un circuit microfluidique. Chaque capsule microfluidique comporte une chambre et une membrane apte à se déformer dans la chambre. Selon sa position, la membrane vient moduler le volume de la chambre. La membrane est actionnée à l'aide de moyens d'actionnement pneumatique. Lorsque la cartouche comporte plusieurs capsules microfluidiques, il est possible de venir utiliser une seule membrane formant une couche spécifique de la cartouche microfluidique. En appliquant une pression ou une dépression localisée sur la membrane, on vient adresser chaque capsule microfluidique de la cartouche. En exerçant une pression positive ou négative sur la membrane, on vient vider ou remplir la chambre en fluide via le circuit microfluidique.

Classiquement, la cartouche est composée de plusieurs couches assemblées entre elles :
- Une couche supérieure qui comporte le circuit microfluidique de la cartouche, le circuit microfluidique pouvant comporter plusieurs éléments fluidiques (chambre, vanne,...).
- La membrane qui est assemblée sur une face de cette couche supérieure.
- Une couche inférieure qui comporte le circuit d'actionnement, en général de type pneumatique.

La couche supérieure et la couche inférieure sont réalisées dans des matériaux polymères de type COC, PMMA ou équivalents.

Les demandes de brevets US2020/206736A1 et US2018/066248A1 décrivent l'intégration d'un insert dans une cartouche microfluidique, l'insert étant mobile en translation pour venir actionner une membrane déformable.

Pour certaines applications, il est également utile de venir chauffer le volume interne de la chambre, par exemple lors d'une réaction d'amplification biomoléculaire. Pour cela, la cartouche est souvent placée sur une platine dotée d'un élément chauffant (Module Peltier, résistance...). Cependant, pour venir chauffer ou refroidir le volume de la chambre, on se heurte à plusieurs contraintes :
- La cartouche est réalisée en matériau de type polymère qui est souvent un très bon isolant thermique. L'élément chauffant doit donc être placé à une température relativement élevée pour venir chauffer suffisamment la chambre.
- La surface disponible sur la cartouche microfluidique est limitée, les différents éléments fluidiques peuvent être donc assez rapprochés sur la surface de la cartouche, rendant difficile un chauffage ou refroidissement localisé d'une chambre en particulier.
- De même, il est difficile d'adresser des éléments fluidiques distincts de la cartouche avec des températures distinctes, le flux de chaleur ou de refroidissement appliqué sur un élément fluidique pouvant se diffuser sur les éléments fluidiques adjacents.

Le but de l'invention est de proposer une solution pour adresser thermiquement de manière précise un élément fluidique d'un circuit microfluidique d'une cartouche mcirofluidique, cet élément fluidique étant réalisé sous la forme d'une capsule microfluidique.

### Exposé de l'invention

Ce but est atteint par une cartouche microfluidique comportant plusieurs couches superposées et assemblées entre elles, lesdites plusieurs couches comportant :
- Une première couche fluidique, dite couche supérieure, réalisée dans un matériau polymère et comprenant un circuit microfluidique, le circuit microfluidique comportant au moins une face, dite inférieure, dans laquelle est réalisée une cavité formant un volume interne ;
- Une deuxième couche comportant une membrane assemblée sur la première couche et venant refermer ladite cavité pour former une capsule microfluidique, ladite membrane étant apte à être contrôlée pour se déformer à l'intérieur de ladite cavité ;

- Une troisième couche, dite couche inférieure, appliquée contre la membrane et réalisée dans un matériau polymère et comportant un circuit d'actionnement de ladite membrane, ledit circuit d'actionnement comportant au moins un passage traversant réalisé à l'aplomb de ladite cavité,
- Ladite cartouche comportant un élément formant un insert fixe solidaire de la couche inférieure et venant se loger dans ledit passage traversant, et disposant d'au moins une zone de contact avec ladite membrane, cet insert étant réalisé dans un matériau conducteur thermique.

Selon une réalisation particulière, l'insert est choisi parmi un gel, une pate thermique ou un solide.

Selon une autre réalisation particulière, l'insert est réalisé dans un matériau solide métallique, choisi parmi l'aluminium, le cuivre, le laiton et un mélange de plusieurs de ces matériaux.

Selon une particularité, la couche inférieure comporte une face supérieure située en vis-à-vis de la membrane et une face inférieure opposée à sa face supérieure, la cartouche comportant également au moins un canal d'actionnement comprenant une entrée débouchant du côté de sa face inférieure et une sortie débouchant sur sa face supérieure, en vis-à-vis de la membrane, à l'aplomb de ladite cavité.

Selon une réalisation particulière, le canal d'actionnement est réalisé en périphérie de l'insert.

Selon une autre réalisation particulière, le canal d'actionnement est réalisé à travers l'insert.

L'invention concerne également un dispositif microfluidique comportant une cartouche microfluidique, une platine dotée d'un élément chauffant ou refroidissant, des moyens d'actionnement pneumatique et une unité de contrôle configurée pour contrôler ledit élément chauffant ou refroidissant et lesdits moyens d'actionnement pneumatique, la cartouche microfluidique étant telle que définie ci-dessus et positionnée sur la platine de sorte que son insert soit en vis-à-vis dudit élément chauffant ou refroidissant et son circuit d'actionnement connecté sur les moyens d'actionnement pneumatique.

Selon une particularité, l'insert est en contact physique direct avec l'élément chauffant ou refroidissant.

Selon une autre particularité, l'insert est séparé de l'élément chauffant ou refroidissant par un film.

L'invention concerne également un procédé de commande du dispositif microfluidique tel que défini ci-dessus, ce procédé comportant des étapes de :
- Contrôle des moyens d'actionnement pneumatique pour mettre la membrane en dépression et la plaquer contre la face supérieure de la couche inférieure ;
- Pendant que la membrane est maintenue par dépression, activation de l'élément chauffant ou refroidissant en vue de transmettre un flux de chaleur ou refroidissant à destination de l'insert pour chauffer ou refroidir le volume interne de ladite cavité.

### Brève description des figures

D'autres caractéristiques et avantages vont apparaître dans la description détaillée qui suit faite en regard des dessins annexés dans lesquels :
- Les figures 1A et 1B représentent le système microfluidique conforme à l'invention, selon un premier mode de réalisation, respectivement avec la membrane au repos et la membrane actionnée ;
- Les figures 2A et 2B représentent le système microfluidique conforme à l'invention, selon un deuxième mode de réalisation, respectivement avec la membrane au repos et la membrane actionnée ;
- La figure 3 illustre le procédé de commande du système microfluidique de l'invention ;

### Description détaillée d'au moins un mode de réalisation

Pour la suite de la description, on définit un repère orthonormé X, Y, Z. Les termes « supérieur », « inférieur », « au-dessus » et « au-dessous » sont à considérer en tenant compte de la direction Z, qui est choisie verticale (comme sur les figures).

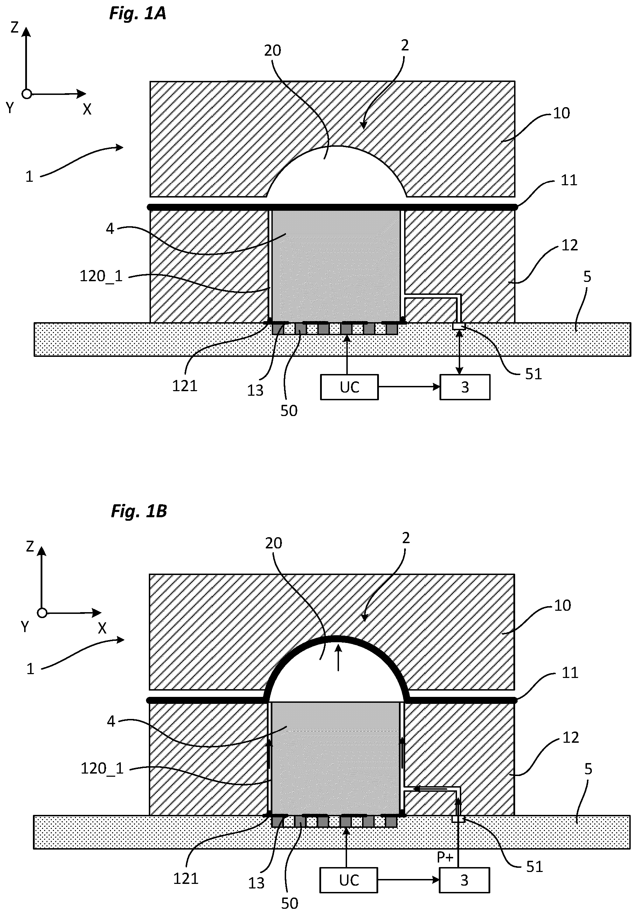

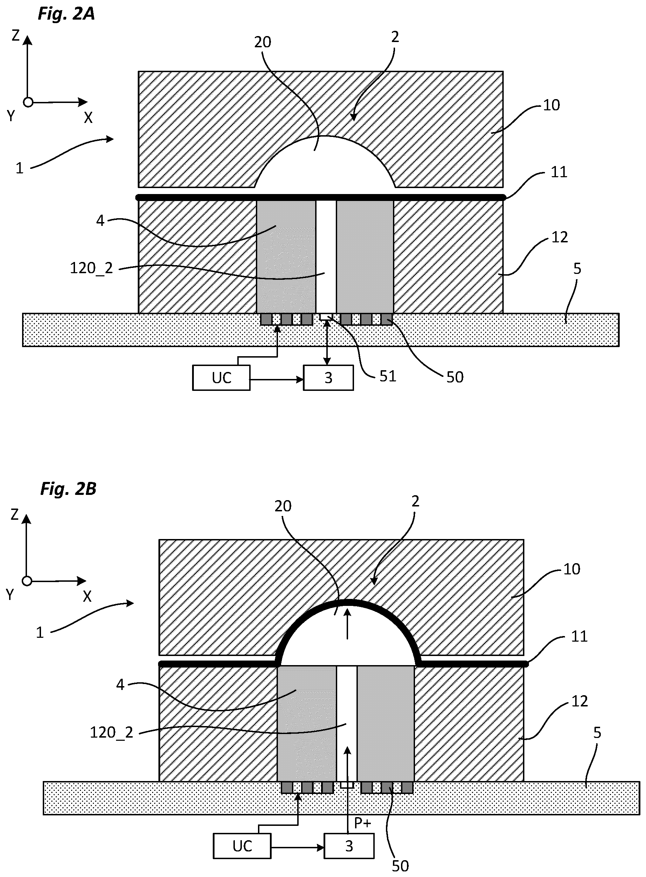

En référence au figures 1A et 1B, et aux figures 2A et 2B, la cartouche microfluidique 1 présente deux faces parallèles opposées s'étendant dans les deux directions X, Y, son épaisseur, faible par rapport aux deux autres dimensions, étant alignée suivant la direction Z.

Suivant la direction Z, la cartouche microfluidique comporte principalement au moins trois couches superposées et assemblées entre elles, ces trois couches étant décrites ci-dessous.

La première couche, qui est la couche supérieure 10, est la couche microfluidique comportant le circuit microfluidique de la cartouche 1. Le circuit microfluidique comporte un ou plusieurs éléments microfluidiques. Un élément microfluidique peut notamment être une capsule microfluidique 2. Sur sa face inférieure, la couche supérieure 10 comporte au moins une cavité 20 destinée à former la capsule microfluidique.

La deuxième couche, appelée également couche intermédiaire, est formée de la membrane 11 appliquée contre la face inférieure de la couche supérieure 10 pour refermer la cavité 20.

La troisième couche est la couche inférieure 12 venant s'appliquer contre la face inférieure de la membrane 11. Cette couche inférieure 12 constitue la couche utilisée pour l'actionnement de la membrane 11 vers l'intérieur de la cavité 20. L'actionnement est avantageusement réalisé de manière pneumatique. Cette couche inférieure 12 comporte ainsi au moins un canal d'actionnement 120_1, 120_2 comportant une entrée venant se connecter sur des moyens d'actionnement pneumatique 3 (capables d'appliquer une pression P+ ou une dépression P-) et une sortie débouchant en vis-à-vis de la membrane 11, à l'aplomb de la cavité 20. On peut imaginer au moins deux solutions d'agencement de ce canal d'actionnement (120_1 sur les figures 1A et 1B ou 120_2 sur les figures 2A et 2B). De manière non limitative, l'entrée du canal d'actionnement 120_1, 120_2 peut être disponible sur la face inférieure de la couche inférieure 12 (comme sur les figures annexées) ou sur une face latérale de la couche inférieure 12.

Selon l'invention, la couche inférieure 12 comporte également un passage traversant sur son toute son épaisseur, ce passage traversant étant réalisé au moins en partie à l'aplomb de ladite cavité 20 de la couche supérieure 10.

Dans ce passage traversant, on vient placer un insert 4 réalisé dans un matériau conducteur thermique. L'insert 4 vient se loger de manière fixe dans le passage traversant et occuper ce passage traversant. L'insert crée au moins une première zone de contact sur sa face supérieure avec la membrane 11 située au-dessus. Du côté inférieur, l'insert 4 présente une deuxième zone de contact sur sa face inférieure, éventuellement refermée par un film 13 (optionnel et illustré en pointillés sur les figures 1A et 1B), destinée à venir s'appuyer contre un élément chauffant ou refroidissant (voir ci-après). Une fois en position dans le passage traversant, l'insert 4 est fixé à la couche inférieure de la cartouche microfluidique 1, par tous moyens adaptés (collage, pâte thermique, emboîtement...) et/ou maintenu en position à l'aide du film 13 précité.

Il faut noter que :
- La couche supérieure 10 et la couche inférieure 12 sont réalisées dans un matériau polymère (avantageusement identique pour les deux couches). Il peut s'agir d'un matériau de type COC, PMMA ou équivalent.
- L'insert 4 est réalisé dans un matériau thermiquement conducteur. Il peut s'agir d'un gel, d'une pâte thermique ou d'un matériau solide. Le matériau solide est avantageusement un matériau métallique, par exemple choisi parmi l'aluminium, le cuivre, le laiton et un mélange de plusieurs de ces matériaux.
- L'insert 4 vient avantageusement occuper toute l'épaisseur de la couche inférieure 12 et est avantageusement réalisé en une seule pièce.
- L'insert 4 peut disposer d'une section identique à la section la plus large de la cavité 20 ou une section élargie par rapport à celle-ci.
- Le film 13 mince appliqué contre la face inférieure de la couche inférieure 12 peut venir recouvrir le passage traversant pour enfermer l'insert 4 du côté inférieur de la cartouche mais il peut également être ajouré pour laisser accessible la deuxième zone de contact de l'insert 4.
- Le film 13 mince est par exemple un adhésif que l'on découpe à la forme voulue par les procédés standards (découpe laser etc...)
- Plusieurs inserts 4 peuvent être intégrés dans la couche inférieure 12, séparés entre eux et destinés chacun à adresser thermiquement un élément fluidique distinct de la cartouche 1.
- Un même insert peut être commun à plusieurs éléments microfluidiques de la cartouche microfluidique.
- L'insert 4 est fixe dans la cartouche microfluidique, solidaire de la cartouche 1 et logé à demeure dans celle-ci. De cette manière la cartouche 1 peut rester un élément consommable et éventuellement jetable ou réutilisable.
- L'insert 4 n'a pas de fonction d'actionnement et de mise en mouvement de la membrane 11.

Sur la figure 1A et la figure 1B, le canal d'actionnement 120_1 ménagé dans la couche inférieure 12 de la cartouche 1 est par exemple agencé en périphérie de l'insert 4. Un joint torique 121 peut être prévu en périphérie de l'insert 4 pour étanchéifier la connexion pneumatique.

Sur la figure 2A et la figure 2B, le canal d'actionnement 120_2 est percé directement à travers l'insert 4 suivant Z, l'insert 4 pouvant présenter une forme annulaire régulière ou irrégulière.

D'autres agencements du canal d'actionnement peuvent être imaginés.

Selon l'invention, en référence aux figures annexées, la cartouche microfluidique 1 est destinée à être positionnée sur une platine 5 du dispositif microfluidique. Cette platine 5 comporte un élément 50 chauffant ou refroidissant intégré et alimenté électriquement. L'élément 50 chauffant ou refroidissant peut être une résistance chauffante, un module Peltier, un échangeur thermique (par exemple avec un fluide frigorigène) ou tout autre élément capable de générer un flux de chaleur ou refroidissant.

La platine 5 peut également comporter un ou plusieurs points d'actionnement 51 pneumatique venant se connecter sur chaque entrée pneumatique de la cartouche. Ces points d'actionnement pneumatique sont reliés aux moyens d'actionnement pneumatique 3 du dispositif.

Le dispositif microfluidique intègre avantageusement une unité de contrôle UC chargée de contrôler les moyens d'actionnement pneumatique 3 et l'élément 50 chauffant ou refroidissant.

Il faut noter que l'insert 4 peut dépasser de la face inférieure de la couche inférieure 12, afin de mieux venir en contact avec l'élément 50 chauffant ou refroidissant situé au-dessous lorsque la cartouche 1 est positionnée sur la platine 5.

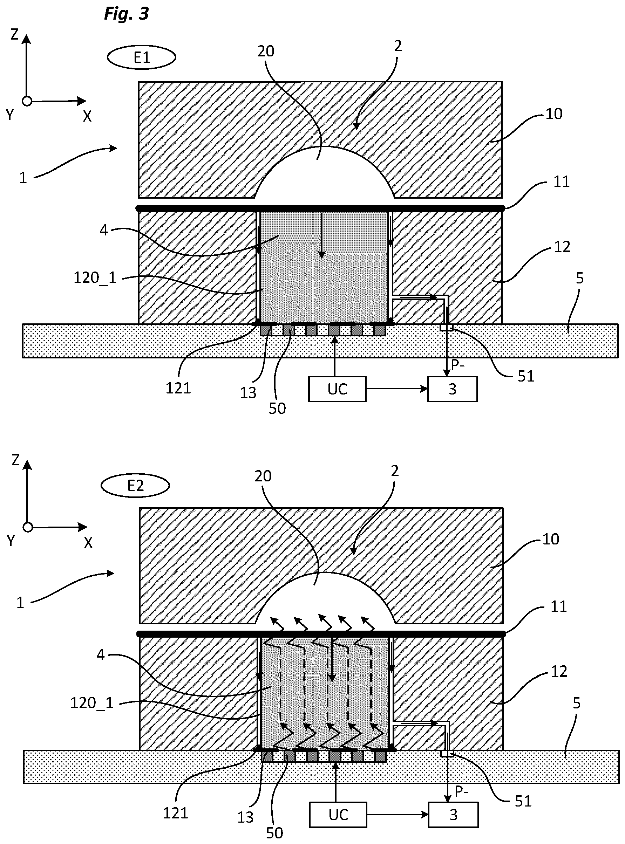

La figure 3 montre les différentes étapes de mise en œuvre du procédé de commande de l'invention. Ce procédé vise à améliorer le transfert thermique à destination du volume interne de la cavité 20 de la capsule microfluidique 2.

Etape E1 : L'unité de contrôle UC active les moyens d'actionnement pneumatique 3 en vue d'appliquer une pression négative (P-) sur le point d'actionnement 51 de la capsule microfluidique 2. La membrane 11 est ainsi actionnée en aspiration et vient se plaquer contre la face supérieure de la couche inférieure 12 de la cartouche 1 et ainsi contre la première zone de contact de l'insert 4.

Etape E2 : Pendant que la membrane 11 est maintenue plaquée par dépression, l'unité de contrôle UC active l'élément 50 chauffant ou refroidissant pour venir chauffer ou refroidir le volume interne de la cavité 20 de la capsule microfluidique 2. Un chauffage d'un échantillon stocké dans le volume interne de la chambre est par exemple réalisé lors de la mise en œuvre d'une réaction d'amplification biomoléculaire (par exemple Polymerase Chain Reaction - PCR).

Selon l'invention, en plaquant la membrane 11 contre la face supérieure de la couche inférieure 12, on vient supprimer la lame d'air présente entre la membrane 11 et la couche inférieure 12 et appliquer la membrane 12 directement contre la première zone de contact de l'insert 4, améliorant ainsi le transfert thermique de l'élément 50 chauffant vers le volume interne de la cavité 20 de la capsule microfluidique 2. La présence d'air peut avoir un effet isolant thermique.

Il est également possible de prévoir un système employant deux capsules microfluidiques adjacentes et reliées entre elles par un canal microfluidique, chaque capsule étant associée à un insert thermique distinct. On peut ainsi déplacer un fluide entre les deux capsules chauffées à des températures distinctes, pour effectuer des cyclages thermiques très rapides et obtenir un gain de temps pour les protocoles comme la PCR

L'invention présente ainsi de nombreux avantages, parmi lesquels :
- Une solution améliorée pour obtenir un chauffage d'une capsule microfluidique ;
- L'insert peut rester solidaire de la cartouche, permettant de rendre la cartouche totalement indépendante de la platine ;
- Une solution qui permet d'adresser thermiquement chaque capsule d'une cartouche microfluidique avec une température cible distincte, même lorsque les capsules sont assez proches les unes des autres au sein de la cartouche ;
- Une solution qui permet de conserver un principe classique d'actionnement, par exemple par des moyens pneumatiques ;

## Revendications

1. Cartouche microfluidique (1) comportant plusieurs couches superposées et assemblées entre elles, lesdites plusieurs couches comportant :
- Une première couche fluidique, dite couche supérieure (10), réalisée dans un matériau polymère et comprenant un circuit microfluidique, le circuit microfluidique comportant au moins une face, dite inférieure, dans laquelle est réalisée une cavité (20) formant un volume interne ;
- Une deuxième couche comportant une membrane (11) assemblée sur la première couche et venant refermer ladite cavité (20) pour former une capsule microfluidique, ladite membrane (11) étant apte à être contrôlée pour se déformer à l'intérieur de ladite cavité ;
- Une troisième couche, dite couche inférieure (12), appliquée contre la membrane et réalisée dans un matériau polymère et comportant un circuit d'actionnement de ladite membrane, ledit circuit d'actionnement comportant au moins un passage traversant réalisé à l'aplomb de ladite cavité (20),
- **Caractérisé en ce que** :
- Ladite cartouche comporte un élément formant un insert (4) fixe solidaire de la couche inférieure (12) et venant se loger dans ledit passage traversant, et disposant d'au moins une zone de contact avec ladite membrane (11), cet insert (4) étant réalisé dans un matériau conducteur thermique.

2. Cartouche selon la revendication 1, **caractérisé en ce que** l'insert (4) est choisi parmi un gel, une pate thermique ou un solide.

3. Cartouche selon la revendication 1, **caractérisé en ce que** l'insert (4) est réalisé dans un matériau solide métallique, choisi parmi l'aluminium, le cuivre, le laiton et un mélange de plusieurs de ces matériaux.

4. Cartouche selon l'une des revendications 1 à 3, **caractérisé en ce que** la couche inférieure (12) comporte une face supérieure située en vis-à-vis de la membrane (11) et une face inférieure opposée à sa face supérieure, et **en ce qu'**elle comporte au moins un canal d'actionnement (120_1, 120_2) comportant une entrée débouchant du côté de sa face inférieure et une sortie débouchant sur sa face supérieure, en vis-à-vis de la membrane (11), à l'aplomb de ladite cavité (20).

5. Cartouche selon l'une des revendications 1 à 4, **caractérisé en ce que** le canal d'actionnement (120_1) est réalisé en périphérie de l'insert (4).

6. Cartouche selon l'une des revendications 1 à 4, **caractérisé en ce que** le canal d'actionnement (120_2) est réalisé à travers l'insert (4).

7. Dispositif microfluidique comportant une cartouche microfluidique (1), une platine (5) dotée d'un élément (50) chauffant ou refroidissant, des moyens d'actionnement pneumatique (3) et une unité de contrôle (UC) configurée pour contrôler ledit élément (50) chauffant ou refroidissant et lesdits moyens d'actionnement pneumatique (3), **caractérisé en ce que** la cartouche microfluidique (1) est telle que définie dans l'une des revendications 1 à 6 et **en ce que** la cartouche microfluidique est positionnée sur la platine (5) de sorte que son insert (4) soit en vis-à-vis dudit élément chauffant ou refroidissant et son circuit d'actionnement connecté sur les moyens d'actionnement pneumatique (3).

8. Dispositif selon la revendication 7, **caractérisé en ce que** l'insert (4) est en contact physique direct avec l'élément (50) chauffant ou refroidissant.

9. Dispositif selon la revendication 7, **caractérisé en ce que** l'insert est séparé de l'élément chauffant ou refroidissant par un film (13).

10. Procédé de commande du dispositif microfluidique tel que défini dans l'une des revendications 7 à 9, **caractérisé en ce qu'**il comporte des étapes de :
- Contrôle des moyens d'actionnement pneumatique (3) pour mettre la membrane (11) en dépression et la plaquer contre la face supérieure de la couche inférieure (12) ;
- Pendant que la membrane (11) est maintenue par dépression, activation de l'élément (50) chauffant ou refroidissant en vue de transmettre un flux de chaleur ou refroidissant à destination de l'insert (4) pour chauffer ou refroidir le volume interne de ladite cavité (20).
